# EUROPÄISCHE PATENTSCHRIFT

(11) **EP 4 142 920 B1**
(45) Veröffentlichungstag und Bekanntmachung des Hinweises auf die Patenterteilung: **28.01.2026**
(21) Anmeldenummer: 21722820.4
(22) Anmeldetag: 28.04.2021
(51) Int. Cl.: B01D 63/08, C12M 1/00, G01N 1/00, C12Q 1/24

(54) **FILTRATIONSEINHEIT, VERFAHREN ZU IHRER HERSTELLUNG, VERFAHREN ZUM NACHWEIS VON MIKROORGANISMEN UND VERWENDUNG DER FILTRATIONSEINHEIT**
FILTRATION UNIT, METHOD FOR PRODUCING SAME, METHOD FOR DETECTING MICROORGANISMS AND USE OF THE FILTRATION UNIT
UNITÉ DE FILTRATION, SON PROCÉDÉ DE PRODUCTION, PROCÉDÉ DE DÉTECTION DE MICRO-ORGANISMES, ET UTILISATION DE L'UNITÉ DE FILTRATION

(30) Priorität: 29.04.2020 DE 102020002588
(43) Veröffentlichungstag der Anmeldung: 08.03.2023
(73) Patentinhaber: Sartorius Stedim Biotech GmbH, 37079 Göttingen (DE)
(72) Erfinder: PFLANZ, Karl, 37079 Göttingen (DE); SCHOLZ, Stephan, 37079 Göttingen (DE); HOSCH, Jörg, 37079 Göttingen (DE)
(74) Vertreter: Novagraaf International SA
(86) Internationale Anmeldenummer: PCT/EP2021/061175
(87) Internationale Veröffentlichungsnummer: WO 2021/219745

(56) Entgegenhaltungen:
- US-A1- 2013 089 890
- US-A1- 2020 078 782

## Beschreibung

Die vorliegende Erfindung betrifft eine Filtrationseinheit mit Membran, Nährkartonscheibe und Stützstruktur, wobei die Nährkartonscheibe und/oder die Stützstruktur ein festes wasserlösliches Nährmedium sowie ein wasserlösliches und/oder wasserquellbares Polymer umfasst, wobei die Nährkartonscheibe, sofern sie das feste wasserlösliche Nährmedium sowie das wasserlösliche und/oder wasserquellbare Polymer umfasst, ein Faservlies aufweist, und das feste wasserlösliche Nährmedium sowie das wasserlösliche und/oder wasserquellbare Polymer entweder
a) die Fasern des Faservlieses teilweise oder vollständig umhüllen, ohne die Poren des Faservlieses zu verschließen, oder
b) als Teilchen zwischen den Fasern verteilt sind, und
wobei die Stützstruktur ein Trägerelement und eine Seitenwand, welche einen Hohlraum definieren, aufweist, die Nährkartonscheibe und die Filtrationsmembran in dem Hohlraum angeordnet sind und die Nährkartonscheibe zwischen der Filtrationsmembran und dem Trägerelement angeordnet ist, und das feste wasserlösliche Nährmedium sowie das wasserlösliche und/oder wasserquellbare Polymer, sofern von der Stützstruktur umfasst, auf das Trägerelement und/oder die Seitenwand aufgebracht sind, ein Verfahren zur Herstellung der Filtrationseinheit, sowie ein Verfahren zum Nachweis von Mikroorganismen in einem Fluid, worin die Filtrationseinheit verwendet wird.

Eine Möglichkeit, wie beispielsweise wässrige Lösungen auf die Gegenwart von Mikroorganismen getestet werden können, ist die sogenannte Membranfiltermethode. Hierbei wird die wässrige Lösung durch einen Membranfilter filtriert, wobei der Membranfilter die gegebenenfalls in der wässrigen Lösung enthaltenen Mikroorganismen zurückhält. Nach der Filtration ist die wässrige Lösung von den Mikroorganismen befreit. Diese befinden sich nunmehr auf bzw. in dem Membranfilter. Nach dem Filtrieren wird der Membranfilter aus der hierzu verwendeten Vorrichtung - beispielsweise mit Hilfe einer sterilen Pinzette - entnommen und auf eine Nährkartonscheibe (NKS) aufgebracht. (Anstelle einer NKS kann auch ein Agar-Medium verwendet werden.) Übliche Nährkartonscheiben umfassen ein poröses Material, beispielsweise ein Faservlies aus Cellulose, und sind mit einem für Mikroorganismen geeigneten Nährmedium imprägniert. Vor dem Aufbringen des Filtermediums auf die NKS wird diese üblicherweise mit Wasser befeuchtet. Dadurch wird das in der NKS enthaltene Nährmedium gelöst, damit die gegebenenfalls auf/in dem Membranfilter befindlichen Mikroorganismen mit dem Nährmedium versorgt werden und wachsen können.

Durch das Inkubieren des befeuchteten Systems aus NKS und Membranfilter wachsen aus den gegebenenfalls eingangs in dem Fluid vorhandenen koloniebildenden Einheiten (CFU) von Mikroorganismen Kolonien heran. Nach dem Inkubieren kann eine Auswertung beispielsweise durch manuelles Zählen der Kolonien erfolgen.

Vorstehende Membranfiltermethode ist jedoch mit mehreren Nachteilen verbunden. So muss die Membran nach der Filtration aus der Filtrationsvorrichtung (beispielsweise eine Nutsche mit Saugflasche) händisch entfernt und auf die NKS aufgebracht werden. Dadurch ist dieses Verfahren nicht nur mit einem hohen Arbeitsaufwand verbunden, sondern weist auch ein hohes Kontaminationsrisiko durch das Transferieren des Membranfilters von der Filtrationsvorrichtung zur NKS auf. Durch Kontaminationen in diesem Schritt können fehlerhafte Ergebnisse erhalten werden.

Um den erforderlichen Arbeitsaufwand und das Kontaminationsrisiko der vorstehend beschriebenen klassischen Membranfiltermethode zu verringern, kann der Schritt der Membranfiltration mit einer Filtereinheit, umfassend eine Filtrationsmembran und ein darunter liegendes Faservlies, durchgeführt werden. Wie auch im klassischen Membranfilterverfahren werden gegebenenfalls im filtrierten Fluid enthaltene Mikroorganismen von der Membran zurückgehalten. Nachdem die Filtration des Fluids beendet ist, wird ein flüssiges Nährmedium auf die Membran aufgebracht. Durch kurzzeitiges Anwenden von Vakuum (mit Hilfe dessen auch die vorangegangene Filtration durchgeführt werden kann) verteilt sich das Nährmedium in der Membran und im Faservlies. Direkt nach diesem Schritt kann die Inkubation erfolgen, gefolgt von der Auswertung. Dieses Verfahren ist als Biosart^{®}-Technologie bekannt.

Bei vorstehend beschriebenem Verfahren muss die Membran vor dem Inkubieren nicht von der Filtrationsvorrichtung zu einer Nährkartonscheibe transportiert werden, wodurch die Gefahr von Kontaminationen verringert ist, wobei hier jedoch durch das händisch erfolgende Aufbringen des Nährmediums ein gewisses Kontaminationsrisiko bestehen bleibt. Zudem besteht bei diesem Verfahren eine Fehlerquelle darin, dass nach dem Aufbringen des flüssigen Nährmediums das Vakuum zu lange angewandt wird, wodurch das Nährmedium durch Membran und Faservlies gesaugt wird. Bei einem Auftreten dieses Fehlers steht für die gegebenenfalls vorhandenen Mikroorganismen im Inkubationsschritt kein Nährmedium zur Verfügung. Werden die Mikroorganismen nicht ausreichend mit Nährmedium versorgt, können sie nicht wachsen, was zu einem fehlerhaften Ergebnis führt. US 2020/078782 offenbart eine Einheit zum Nachweis von Mikro-Organismen mit integriertem Filter 32, d.h. einer Membran, einer Nährkartonscheibe und Träger (48,49), d.h. ein Trägerelement. Die Nährkartonscheibe ist ein Absorbens, wie z.B. Rayon, Baumwolle, natürliche oder chemisch modifizierte Cellulose Fasern, oder ein Absorbens aus Polyacrylat oder Acrylat- acrylamid-copolymer. US 2013/089890 offenbart ebenfalls eine Einheit zum Nachweis von Mikroorganismen, wobei der Filter nach der Filtration in einem separaten Schritt mit dem Nährmedium in Kontakt gebracht wird.

Der vorliegenden Erfindung liegt somit die Aufgabe zugrunde, ein Verfahren zum Nachweis von Mikroorganismen in einem Fluid, welches eine niedrige Nachweisgrenze aufweist sowie einfach durchgeführt werden kann und welches mit einer geringen Kontaminationsgefahr einhergeht; eine Filtrationseinheit, welche die Durchführung des Verfahrens ermöglicht, und ein Verfahren zur Herstellung der Filtrationseinheit, bereitzustellen.

Die vorstehende Aufgabe wird durch die in den Ansprüchen gekennzeichneten Ausführungsformen gelöst.

In einem ersten Aspekt betrifft die vorliegende Erfindung eine Filtrationseinheit, umfassend eine Filtrationsmembran, eine Nährkartonscheibe und eine Stützstruktur, wobei zumindest eine von der Nährkartonscheibe und der Stützstruktur ein festes wasserlösliches Nährmedium sowie ein wasserlösliches und/oder wasserquellbares Polymer umfasst, wobei die Nährkartonscheibe, sofern sie das feste wasserlösliche Nährmedium sowie das wasserlösliche und/oder wasserquellbare Polymer umfasst, ein Faservlies aufweist, und das feste wasserlösliche Nährmedium sowie das wasserlösliche und/oder wasserquellbare Polymer entweder
a) die Fasern des Faservlieses teilweise oder vollständig umhüllen, ohne die Poren des Faservlieses zu verschließen, oder
b) als Teilchen zwischen den Fasern verteilt sind, und
wobei die Stützstruktur ein Trägerelement und eine Seitenwand, welche einen Hohlraum definieren, aufweist, die Nährkartonscheibe und die Filtrationsmembran in dem Hohlraum angeordnet sind und die Nährkartonscheibe zwischen der Filtrationsmembran und dem Trägerelement angeordnet ist, und das feste wasserlösliche Nährmedium sowie das wasserlösliche und/oder wasserquellbare Polymer, sofern von der Stützstruktur umfasst, auf das Trägerelement und/oder die Seitenwand aufgebracht sind.

Die erfindungsgemäße Filtrationseinheit stellt eine Weiterentwicklung der Biosart^{®}-Technologie dar. Erfindungsgemäß liegt das wasserlösliche Nährmedium bereits vor der Filtration vor. Somit muss es nicht nach der Filtration zugegeben werden. Die Gegenwart des wasserlöslichen bzw. wasserquellbaren Polymers führt zu einem verzögerten Auflösen des Nährmediums in dem Fluid und verhindert somit das vollständige Auswaschen des Nährmediums im Filtrationsschritt. Mit der erfindungsgemäßen Filtrationseinheit können besonders einfach sowie mit niedriger Nachweisgrenze und niedrigem Kontaminationsrisiko Mikroorganismen in einem Fluid nachgewiesen werden.

Erfindungsgemäß wird unter einer "Filtrationseinheit" ein Erzeugnis verstanden, welches zur Filtration verwendet werden kann. Bevorzugt liegt die Filtrationseinheit in steriler Form vor.

Das wasserlösliche bzw. wasserquellbare Polymer unterliegt keiner besonderen Einschränkung, abgesehen davon, dass es in Wasser bzw. einem wässrigen Medium löslich beziehungsweise quellbar ist und keine abtötende bzw. wachstumshemmende Wirkung auf die untersuchten Mikroorganismen aufweist.

Hierin wird ein Polymer als "wasserlöslich" betrachtet, sofern bei 25°C mindestens 1 g/L, vorzugsweise 10 g/L, insbesondere bevorzugt 100 g/L des Polymers in Wasser gelöst werden können.

Hierin wird ein Polymer als "wasserquellbar" betrachtet, sofern es bei 25°C mindestens 100 Gew.-%, vorzugsweise mindestens 500 Gew.-%, besonders bevorzugt mindestens 1000 Gew.-%, seines Trockengewichts an Wasser aufnehmen kann und durch die Wasseraufnahme eine Volumenerhöhung erfährt, ohne von dem Wasser gelöst zu werden. Vorzugsweise ist das wasserquellbare Polymer ein vernetztes hydrophiles Polymer.

Gemäß einer bevorzugten Ausführungsform der vorliegenden Erfindung ist das wasserlösliche Polymer aus der Gruppe, bestehend aus Polyvinylpyrrolidon, Gelatine, Agarose, sowie Gemischen daraus, ausgewählt. Es ist besonders bevorzugt, dass das wasserlösliche Polymer Polyvinylpyrrolidon enthält bzw. aus diesem besteht.

Gemäß einer bevorzugten Ausführungsform der vorliegenden Erfindung weist das Polyvinylpyrrolidon einen K-Wert nach Fikentscher von 20 bis 200, vorzugsweise von 25 bis 120, insbesondere bevorzugt von 30 bis 100, noch bevorzugter von 60 bis 90, auf.

Der auch als Eigenviskosität bezeichnete K-Wert wird über Viskositätsmessungen von Polymer-Lösungen bestimmt und häufig im technischen Bereich zur Bestimmung der molaren Masse von Polymeren verwendet. Der K-Wert ist abhängig von der mittleren molaren Masse der untersuchten Polymere.

Erfindungsgemäß kann der K-Wert wie folgt bestimmt werden. Das Prinzip der Bestimmungsmethode beruht auf der kapillarviskosimetrischen Bestimmung der relativen Lösungsviskosität. Hierzu wird die Testsubstanz in Toluol durch dreißigminütiges Schütteln aufgelöst, so dass man eine 1 Gew.-%-ige Lösung erhält. In einem Vogel-Ossag-Viskosimeter wird bei 25 °C die Auslaufzeit gemessen und daraus in Bezug auf die Viskosität des reinen Lösungsmittels die relative Viskosität der Probenlösung bestimmt. Aus Tabellen kann nach Fikentscher [P. E. Hinkamp, Polymer, 1967, 8, 381] der K-Wert abgelesen werden (K = 1000 k).

Gemäß einer bevorzugten Ausführungsform der vorliegenden Erfindung ist das wasserquellbare Polymer aus der Gruppe, bestehend aus vernetztem Polyvinylpyrrolidon, vernetztem Polyvinylacetat, Polyacrylat, Polymethacrylat, Acrylat-Methacrylat-Copolymer, sowie Gemischen daraus ausgewählt.

Erfindungsgemäß kann die Nährkartonscheibe beziehungsweise die Stützstruktur ein wasserlösliches Polymer, ein wasserquellbares Polymer oder sowohl ein wasserlösliches Polymer als auch ein wasserquellbares Polymer umfassen beziehungsweise enthalten.

Die verwendete Filtrationsmembran unterliegt erfindungsgemäß keiner besonderen Einschränkung. Grundsätzlich können alle Filtrationsmembranen, welche für die vorstehend beschriebenen Verfahren aus dem Stand der Technik geeignet sind, auch in der vorliegenden Erfindung Einsatz finden. Gängige Filtrationsmembranen bestehen beispielsweise aus Nitrocellulose. Dabei wird die Porengröße der Filtrationsmembran so gewählt, dass die Mikroorganismen, auf die abgestellt wird, zurückgehalten werden und andere Bestandteile des untersuchten Fluids die Membran (sowie auch die NKS) passieren können.

Auch das feste wasserlösliche Nährmedium unterliegt erfindungsgemäß keiner besonderen Einschränkung. Geeignete Nährmedien sind dem Fachmann hinlänglich bekannt. Grundsätzlich sind die aus dem Stand der Technik bekannten wasserlöslichen Nährmedien auch für die vorliegende Erfindung geeignet.

Erfindungsgemäß wird unter einer "Nährkartonscheibe" (NKS) eine flächige (bevorzugt scheibenförmige) poröse Struktur verstanden, wobei gegebenenfalls in den Poren der Nährkartonscheibe ein Nährmedium verteilt ist, ohne die Poren zu verschließen. Die Poren der Nährkartonscheibe sind vorzugsweise um ein Vielfaches größer als die Poren der Filtrationsmembran. In diesem Fall wird die Filtrationswirkung (Zurückhalten von gegebenenfalls enthaltenen Mikroorganismen) durch die Filtrationsmembran erzielt, wohingegen die poröse Struktur der Nährkartonscheibe lediglich dazu dient, einen Vorrat des Nährmediums bereitzustellen. Hierbei kann die poröse Struktur der Nährkartonscheibe auf unterschiedliche Weise gebildet sein, beispielsweise durch eine schwammartige oder faserförmige Trägerstruktur, wobei eine faserförmige Trägerstruktur bevorzugt ist.

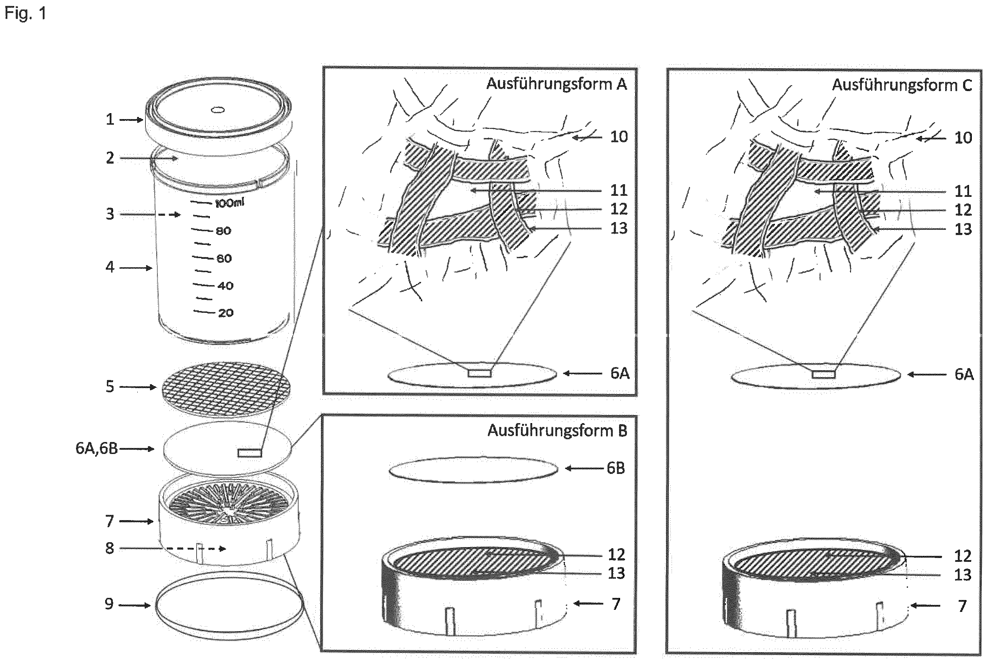

Wie vorstehend beschrieben, wird erfindungsgemäß unter "Nährkartonscheibe" eine Struktur verstanden, wobei gegebenenfalls in den Poren der Nährkartonscheibe ein Nährmedium verteilt ist. Somit umfasst die allgemeinste Definition der Nährkartonscheibe eine Kartonscheibe für Nährmedium, auf die das Nährmedium (noch) nicht aufgebracht worden ist. Eine entsprechende Ausführungsform ist in der nachstehend beschriebenen Figur 1 mit dem Bezugszeichen 6B beschrieben.

Die erfindungsgemäße NKS liegt bevorzugt in steriler Form vor. Erfindungsgemäß besonders bevorzugt liegen die NKS und die Filtrationsmembran in steriler Form vor.

Gemäß einer bevorzugten Ausführungsform der vorliegenden Erfindung schließt die Nährkartonscheibe ein Faservlies ein. Somit weist in diesem Fall die NKS ein Faservlies sowie gegebenenfalls ein festes wasserlösliches Nährmedium und ein wasserlösliches und/oder wasserquellbares Polymer auf. Das Nährmedium und das Polymer, sofern vorhanden, umhüllen (überziehen, beschichten) die Fasern des Faservlieses teilweise oder vollständig, ohne die Poren des Faservlieses zu verschließen. Daneben bzw. alternativ hierzu ist das feste wasserlösliche Nährmedium zusammen mit dem wasserlöslichen und/oder wasserquellbaren Polymer, sofern vorhanden, als Teilchen zwischen den Fasern verteilt.

Gemäß einer bevorzugten Ausführungsform der vorliegenden Erfindung umfasst die NKS, die hier auch als Kartonscheibe bezeichnet werden kann, ein Faservlies, wobei kein festes wasserlösliches Nährmedium und kein wasserlösliches und/oder wasserquellbares Polymer vorliegt.

In einer bevorzugten Ausführungsform der vorliegenden Erfindung umfasst (beziehungsweise enthält) die Nährkartonscheibe das feste wasserlösliche Nährmedium sowie das wasserlösliche und/oder wasserquellbare Polymer.

In einer weiteren bevorzugten Ausführungsform der vorliegenden Erfindung umfasst die Stützstruktur das feste wasserlösliche Nährmedium sowie das wasserlösliche und/oder wasserquellbare Polymer.

Sofern die Stützstruktur das Nährmedium und das Polymer umfasst, ist nicht erforderlich, dass auch die NKS Nährmedium und Polymer umfasst. Sie kann in diesem Fall beispielsweise aus einem Faservlies bestehen. Es ist gemäß der vorliegenden Erfindung jedoch auch möglich, dass sowohl die Stützstruktur als auch die NKS das feste wasserlösliche Nährmedium sowie das wasserlösliche und/oder wasserquellbare Polymer umfassen, beziehungsweise dass nur die NKS Nährmedium und Polymer umfasst.

Es ist bevorzugt, dass das Faservlies der NKS aus Cellulosefasern aufgebaut ist bzw. aus Cellulosefasern besteht. Es sind jedoch auch andere Fasermaterialien geeignet. So kann das Faservlies der NKS auch aus Polyvinylacrylat-Fasern (PVA-Fasern) aufgebaut sein bzw. daraus bestehen.

Gemäß einer weiteren bevorzugten Ausführungsform ist die Nährkartonscheibe ein Faservlies, dessen Fasern teilweise oder vollständig mit einem ersten Überzug, gebildet von dem Nährmedium, und einem zweiten Überzug, gebildet von dem wasserlöslichen und/oder wasserquellbaren Polymer und aufgebracht auf dem ersten Überzug, gebildet ist. Durch diese besondere Struktur wird im Filtrationsschritt höchstens ein geringer Anteil des Nährmediums ausgewaschen.

Gemäß der vorliegenden Erfindung weist die Stützstruktur ein Trägerelement und eine Seitenwand auf, welche einen Hohlraum definieren. Hierbei sind die Nährkartonscheibe und die Filtrationsmembran in dem Hohlraum angeordnet und die Nährkartonscheibe ist zwischen der Filtrationsmembran und dem Trägerelement angeordnet. Vorzugsweise ist der Hohlraum nach einer Raumrichtung hin geöffnet. Besonders bevorzugt schließt die Stützstruktur die Gestalt eines nach einer Seite hin offenen Zylinders ein, wobei sich das Trägerelement an der Seite befindet, welche der offenen Seite gegenübersteht, und die Seitenwand die Gestalt eines beidseitig offenen Zylinders einnimmt.

Unter vorstehenden und nachstehenden Zylindern (Seitenwand, Trägerelement) wird erfindungsgemäß grundsätzlich ein geometrischer Körper verstanden, bei dem zwei parallele, ebene, virtuelle (da offener Zylinder) Grundflächen durch einen Mantel miteinander verbunden sind. Die Grundflächen können grundsätzlich jede Form wie beispielsweise viereckig, rechteckig, quadratisch, polygonal, kreisförmig und dreieckig aufweisen. Die obere und untere (virtuelle) Grundfläche können gleich (kongruent) oder voneinander verschieden sein, wie beispielsweise bei einer Trichterform. Gemäß bevorzugter Ausführungsformen stellen die Zylinder (Seitenwand, Trägerelement) jeweils einen geometrischen Körper dar, bei dem zwei parallele, ebene, kongruente, kreisrunde virtuelle Grundflächen durch einen Mantel miteinander verbunden sind.

Es ist erfindungsgemäß bevorzugt, dass die Seitenwand in Form eines beidseitig offenen Zylinders vorliegt, welcher an einer offenen Seite einen Einlass und an der gegenüberliegenden offenen Seite des Einlasses der Seitenwand einen Auslass aufweist, wobei sich, ausgehend von der Nährkartonscheibe, der Einlass der Seitenwand in Richtung der Filtrationsmembran und der Auslass der Seitenwand in Richtung des Trägerelements befindet. Ohne hierauf erfindungsgemäß beschränkt zu sein, kann der Einlass der Seitenwand eine Trichterform aufweisen, welche sich in Richtung der Seitenwand verjüngt. Der Einlass der Seitenwand kann hierbei erfindungsgemäß dem Einlass der Filtrationseinheit entsprechen.

Das Trägerelement und die Seitenwand der Stützstruktur können eine integrale (monolithische, aus einem Stück bestehende) Einheit bilden. Ebenso kann die Stützstruktur durch einen Zusammenbau von Trägerelement und Seitenwand gebildet sein. Die Stützstruktur kann aus dem Trägerelement und der Seitenwand (sowie gegebenenfalls aus Nährmedium und wasserlöslichem und/oder wasserquellbarem Polymer, aufgebracht auf Trägerelement und/oder Seitenwand) bestehen oder zusätzlich noch weitere Elemente/Bauteile aufweisen, wie beispielsweise einen ersten Deckel zum Verschließen der offenen Seite des vorstehend definierten Hohlraums. Eine erfindungsgemäß geeignete Stützstruktur ist Biosart^{®}-Monitor von der Firma Sartorius Stedim GmbH, umfassend eine Basis als Trägerelement, einen Biosart^{®}-Zylinder als Seitenwand sowie einen Deckel.

Das Trägerelement liegt erfindungsgemäß bevorzugt in Form eines beidseitig offenen Zylinders vor, welcher an einer der offenen Seiten des Zylinders einen Einlass und an der gegenüberliegenden offenen Seite des Einlasses des Trägerelements einen Auslass aufweist, wobei sich der Einlass des Trägerelements in Richtung des Hohlraums befindet. Erfindungsgemäß kann hierbei der Einlass des Trägerelements dem Auslass der Seitenwand entsprechen. Weiterhin kann der Auslass des Trägerelements erfindungsgemäß dem Auslass der Filtrationseinheit entsprechen.

Bevorzugt wird der Zusammenbau des Trägerelements mit der Seitenwand zu einer integralen Einheit durch fluiddichtes Verklemmen des Auslasses der Seitenwand mit dem Einlass des Trägerelements mit der dazwischen liegenden Filtrationsmembran in ihren Randbereichen realisiert. Alternativ oder zusätzlich kann die Filtrationsmembran in ihren Randbereichen mit dem Trägerelement versiegelt bzw. verschweißt sein. Erfindungsgemäß ist dabei der äußere Umfang des beidseitig offenen Zylinders der Seitenwand geringer dimensioniert als der innere Umfang des beidseitig offenen Zylinders des Trägerelements.

Es ist erfindungsgemäß bevorzugt, dass der erste Deckel den Einlass der Seitenwand und/oder den Einlass der Filtrationseinheit reversibel verschließen kann. Bevorzugt verschließt ein zweiter Deckel reversibel den Auslass des Trägerelements und/oder den Auslass der Filtrationseinheit. Beide vorstehend definierten Deckel sind erfindungsgemäß nicht weiter beschränkt, sofern sich diese jeweilig zum reversiblen Verschließen des vorstehend definierten Einlasses oder Auslasses eignen. Erfindungsgemäß bevorzugt liegt mindestens einer des ersten und zweiten Deckels, besonders bevorzugt der erste und der zweite Deckel, in steriler Form vor.

Gemäß der vorliegenden Erfindung kann nur die Seitenwand oder nur das Trägerelement das feste wasserlösliche Nährmedium sowie das wasserlösliche und/oder wasserquellbare Polymer umfassen. Es können allerdings auch sowohl die Seitenwand als auch das Trägermedium Nährmedium und Polymer umfassen. Vorzugsweise umfasst die Innenseite der Seitenwand das Nährmedium sowie das wasserlösliche und/oder wasserquellbare Polymer.

Das Trägerelement unterliegt keiner besonderen Einschränkung, abgesehen davon, dass es für Fluide durchlässig ist, um eine Filtration durch die Membran, die Nährkartonscheibe und das Trägerelement zu ermöglichen. Das Trägerelement kann beispielsweise eine Lochplatte, eine Gitterstruktur oder eine Fritte sein. Gemäß einer besonders bevorzugten Ausführungsform ist die Lochplatte, Gitterstruktur oder Fritte innerhalb des vorstehend genannten beidseitig offenen Zylinders senkrecht zur Zylinderachse angeordnet, wobei Lochplatte, Gitterstruktur oder Fritte integral mit der Seitenwand des Zylinders verbunden sind.

Hinsichtlich des Materials des Trägerelements und der Seitenwand besteht keine besondere Einschränkung. Das Trägerelement wie auch die Seitenwand können beispielsweise aus Metall, Kunststoff, Keramik, Glas oder mehreren davon aufgebaut sein. Das Trägerelement und die Seitenwand der Stützstruktur bewirken eine besonders hohe mechanische Stabilität der Filtrationseinheit.

Die Stützstruktur liegt vorzugsweise in steriler Form vor. Besonders bevorzugt liegen erfindungsgemäß die Seitenwand und/oder das Trägerelement steril vor.

Es ist erfindungsgemäß bevorzugt, dass sich die Filtrationsmembran und die NKS miteinander in Kontakt befinden. Das heißt, es ist bevorzugt, dass sich die Filtrationsmembran und die NKS berühren bzw. direkt aneinander angrenzen. Weiterhin ist es bevorzugt, dass sich die NKS und die Stützstruktur (insbesondere das Trägerelement), sofern vorhanden, in Kontakt miteinander befinden (sich berühren/direkt aneinander angrenzen). "Direkt aneinander angrenzen" bedeutet, dass sich zwischen der Filtrationsmembran und der NKS beziehungsweise zwischen der NKS und der Stützstruktur (insbesondere dem Trägerelement) keine weiteren Lagen/Schichten befinden.

Gemäß einer bevorzugten Ausführungsform der vorliegenden Erfindung ist die Filtrationsmembran in ihren Randbereichen fluiddicht mit der Stützstruktur verbunden. "Fluiddicht" bedeutet, dass der mit der Stützstruktur verbundene Randbereich der Filtrationsmembran für Fluide, insbesondere Flüssigkeiten, undurchlässig ist. Durch eine fluiddichte Verbindung kann das unter Umständen mögliche Entstehen von Bypässen bei der Filtration vermieden werden. Die Bildung von Bypässen bei der Filtration ist unerwünscht, da es hierdurch möglich ist, dass die zu detektierenden Mikroorganismen des Fluids über einen Bypass an der Filtrationsmembran vorbeigeführt werden, ohne dass sie von der Filtrationsmembran zurückgehalten werden.

Erfindungsgemäß kann das fluiddichte Verbinden der Randbereiche der Filtrationsmembran mit der Stützstruktur durch fluiddichtes Verklemmen und/oder Versiegeln der Randbereiche der Filtrationsmembran mit der Stützstruktur erreicht werden. Dabei ist die NKS so weit geringer dimensioniert, dass sie nicht in den Randbereich eingreift, der zur fluiddichten Verklemmung und/oder Versiegelung dient.

Durch eine fluiddichte Verbindung wird zudem sichergestellt, dass die Definition des gebildeten Medienvolumens für eine reproduzierbar einstellbare Konzentration der Mikroorganismen sichergestellt ist.

Eine fluiddichte Verbindung der Randbereiche der Filtrationsmembran mit der Stützstruktur hat den Vorteil, dass nach der Filtration keine Luft nachströmen und das Fluidvolumen unter der Membran verändern kann, denn die Passage von Luft durch eine benetzte Membran wird aufgrund des durch den Blasenpunkt definierten Drucks verhindert. Somit dichtet die nach der Filtration benetzte Filtrationsmembran bei einer fluiddichten Verbindung ihrer Randbereiche mit der Stützstruktur gegen nachströmende Luft ab und bildet eine obere Begrenzung des Medienvolumens. Durch zusätzlich erfindungsgemäß bevorzugtes Verschließen des Auslasses der Filtrationseinheit nach dem Filtrationsschritt und vor dem Schritt des Inkubierens kann sichergestellt werden, dass das Medienvolumen durch die Filtrationsmembran, deren mit der Stützstruktur fluiddicht verbundene Randbereiche und die Stützstruktur (gegebenenfalls mit Trägerelement) sicher definiert ist.

Durch den vorstehend definierten erfindungsgemäßen Aufbau der Filtrationseinheit können Mikroorganismen in einem Fluid mit verringertem Kontaminationsrisiko nachgewiesen werden. Damit wird zugleich die Ergebnisverfälschung des Nachweises von Mikroorganismen in einem Fluid minimiert bzw. die Zuverlässigkeit des Nachweises erhöht.

Ohne hierauf erfindungsgemäß beschränkt zu sein ist in Figur 1 beispielhaft eine erfindungsgemäße Filtrationseinheit dargestellt, welche einen ersten reversiblen Deckel 1, einen Einlass 2 der Filtrationseinheit, einen Hohlraum 3, eine Seitenwand 4, eine Filtrationsmembran 5, eine Nährkartonscheibe (NKS) 6A bzw. Kartonscheibe 6B, ein Trägerelement 7, einen Auslass 8 der Filtrationseinheit und einen zweiten reversiblen Deckel 9 aufweist.

Das Trägerelement 7 und die Seitenwand 4 definieren dabei einen Hohlraum 3, die NKS 6A bzw. Kartonscheibe 6B und die Filtrationsmembran 5 sind in diesem Hohlraum 3 übereinander angeordnet und die NKS 6A bzw. Kartonscheibe 6B ist dabei zwischen der Filtrationsmembran 5 und dem Trägerelement 7 angeordnet. Die Seitenwand 4 liegt in Gestalt eines beidseitig offenen Zylinders vor und weist an einer der offenen Seiten einen Einlass auf und an der gegenüberliegenden offenen Seite des Einlasses der Seitenwand 4 einen Auslass auf, wobei sich, ausgehend von der NKS 6A bzw. Kartonscheibe 6B, der Einlass der Seitenwand 4 in Richtung der Filtrationsmembran 5 und der Auslass der Seitenwand 4 in Richtung des Trägerelements 7 befindet. Der Einlass der Seitenwand 4 entspricht hierbei dem Einlass 2 der Filtrationseinheit. Weiterhin ist der Einlass 2 der Filtrationseinheit reversibel mit einem ersten Deckel 1 verschlossen. Die Filtrationsmembran 5 ist in ihren Randbereichen fluiddicht mit dem Auslass der Seitenwand 4 und dem Einlass des Trägerelements 7 verbunden. Die NKS 6A bzw. Kartonscheibe 6B grenzt dabei direkt mit der Filtrationsmembran 5 und dem Trägerelement 7 aneinander.

Das Trägerelement 7 befindet sich am Auslass der Seitenwand 4 und liegt in Form eines beidseitig offenen Zylinders vor, welcher an einer der offenen Seiten einen Einlass aufweist und an der gegenüberliegenden offenen Seite des Einlasses des Trägerelements 7 einen Auslass aufweist, wobei sich der Einlass des Trägerelements 7 in Richtung des Hohlraums 3 befindet. Der Einlass des Trägerelements 7 entspricht hierbei dem Auslass der Seitenwand 4 und der Auslass des Trägerelements 7 entspricht dem Auslass 8 der Filtrationseinheit. Das Trägerelement 7 liegt im Inneren in Form einer für Fluide durchlässigen bzw. porösen Gitterstruktur vor.

Der Zusammenbau des Trägerelements 7 mit der Seitenwand 4 zu einer integralen Einheit wird durch fluiddichtes Verklemmen des Auslasses der Seitenwand 4 mit dem Einlass des Trägerelements 7 mit der dazwischen liegenden Filtrationsmembran 5 in ihren Randbereichen realisiert. Alternativ oder zusätzlich kann die Filtrationsmembran 5 in ihren Randbereichen mit dem Trägerelement 7 versiegelt bzw. verschweißt sein. Dabei ist die NKS 6A bzw. Kartonscheibe 6B so weit geringer dimensioniert, dass diese nicht in den Randbereich eingreift, der zur fluiddichten Verklemmung und/oder Versiegelung dient. Weiterhin ist der Auslass 8 der Filtrationseinheit mit einem zweiten reversiblen Deckel 9 verschlossen. Die komplette Filtrationseinheit liegt in steriler Form vor.

In einer Ausführungsform A dieser vorstehend beispielhaften Filtrationseinheit weist die NKS 6A eine poröse Struktur aus Faservlies 10, offenen Poren 11, Nährmedium 12 und wasserlöslichem und/oder wasserquellbarem Polymer 13 auf. Dabei umhüllen das Nährmedium 12 und das wasserlösliche und/oder wasserquellbare Polymer 13 die Fasern des Faservlieses 10 teilweise bis vollständig, ohne die offenen Poren 11 des Faservlieses 10 zu verschließen. Die poröse Gitterstruktur im Inneren des Trägerelements 7 ist dabei nicht zusätzlich mit dem Nährmedium 12 und/oder dem wasserlöslichen und/oder wasserquellbaren Polymer 13 beschichtet. Hierbei kann die innere Oberfläche der Seitenwand 4 zusätzlich mit dem Nährmedium 12 und dem wasserlöslichen und/oder wasserquellbaren Polymer 13 beschichtet sein.

In einer weiteren Ausführungsform B dieser vorstehend beispielhaften Filtrationseinheit weist die Kartonscheibe 6B eine poröse Struktur aus Faservlies 10 und offenen Poren 11 auf, ohne dass das Faservlies 10 mit dem Nährmedium 12 und/oder dem wasserlöslichem und/oder wasserquellbarem Polymer 13 teilweise bis vollständig umhüllt ist. Die poröse Gitterstruktur im Inneren des Trägerelements 7 und/oder die innere Oberfläche der Seitenwand 4 ist/sind hingegen mit dem Nährmedium 12 und dem wasserlöslichen und/oder wasserquellbaren Polymer 13 beschichtet.

In einer zusätzlichen Ausführungsform C dieser vorstehend beispielhaften Filtrationseinheit weist die NKS 6A eine poröse Struktur aus Faservlies 10, offenen Poren 11, Nährmedium 12 und wasserlöslichem und/oder wasserquellbarem Polymer 13 auf. Dabei umhüllen das Nährmedium 12 und das wasserlösliche und/oder wasserquellbare Polymer 13 die Fasern des Faservlieses 10 teilweise bis vollständig, ohne die offenen Poren 11 des Faservlieses 10 zu verschließen. Die poröse Gitterstruktur im Inneren des Trägerelements 7 und/oder die innere Oberfläche der Seitenwand 4 ist/sind hierbei zusätzlich mit dem Nährmedium 12 und dem wasserlöslichen und/oder wasserquellbaren Polymer 13 beschichtet.

In einem weiteren Aspekt betrifft die vorliegende Erfindung ein Verfahren zur Herstellung der erfindungsgemäßen Filtrationseinheit, welches die folgenden Schritte umfasst: Bereitstellen der Filtrationsmembran; Bereitstellen einer Nährkartonscheibe, welche frei von dem wasserlöslichen Nährmedium sowie dem wasserlöslichen und/oder wasserquellbaren Polymer ist; Bereitstellen einer wässrigen Lösung des wasserlöslichen Nährmediums; Bereitstellen einer Zusammensetzung, enthaltend das wasserlösliche und/oder wasserquellbare Polymer; Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist; Inkontaktbringen der Nährkartonscheibe und/oder der Stützstruktur mit der wässrigen Lösung des wasserlöslichen Nährmediums, gefolgt von Trocknen, wodurch eine Nährkartonscheibe beziehungsweise eine Stützstruktur, die das Nährmedium umfasst, erhalten wird; Inkontaktbringen der Nährkartonscheibe und/oder der Stützstruktur, die das Nährmedium umfasst, mit der Zusammensetzung, enthaltend das wasserlösliche und/oder wasserquellbare Polymer, gefolgt von Trocknen, wodurch die Nährkartonscheibe beziehungsweise die Stützstruktur, die das feste wasserlösliche Nährmedium sowie das wasserlösliche und/oder wasserquellbare Polymer umfassen, erhalten wird; und Anordnen der Filtrationsmembran, der Nährkartonscheibe und der Stützstruktur zur Filtrationseinheit.

Gemäß einer bevorzugten Ausführungsform umfasst das Herstellungsverfahren die folgenden Schritte: Bereitstellen der Filtrationsmembran; Bereitstellen einer Nährkartonscheibe, welche frei von dem wasserlöslichen Nährmedium sowie dem wasserlöslichen und/oder wasserquellbaren Polymer ist; Bereitstellen einer wässrigen Lösung des wasserlöslichen Nährmediums; Bereitstellen einer wässrigen Lösung des wasserlöslichen Polymers; Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist; Inkontaktbringen der Nährkartonscheibe und/oder der Stützstruktur mit der wässrigen Lösung des wasserlöslichen Nährmediums, gefolgt von Trocknen, wodurch eine Nährkartonscheibe beziehungsweise ein Stützstruktur, die das Nährmedium umfasst, erhalten wird; Inkontaktbringen der Nährkartonscheibe und/oder der Stützstruktur, die das Nährmedium umfasst, mit der wässrigen Lösung des wasserlöslichen Polymers, gefolgt von Trocknen, wodurch die Nährkartonscheibe beziehungsweise die Stützstruktur, die das feste wasserlösliche Nährmedium sowie das wasserlösliche Polymer umfassen, erhalten wird; und Anordnen der Filtrationsmembran, der Nährkartonscheibe und der Stützstruktur zur Filtrationseinheit.

Die vorliegende Erfindung betrifft des Weiteren ein Verfahren zur Herstellung der erfindungsgemäßen Filtrationseinheit, welches die folgenden Schritte umfasst: Bereitstellen der Filtrationsmembran; Bereitstellen einer Nährkartonscheibe welche frei von dem wasserlöslichen Nährmedium sowie dem wasserlöslichen und/oder wasserquellbaren Polymer ist; Bereitstellen einer wässrigen Zusammensetzung, enthaltend das wasserlösliche Nährmedium und das wasserlösliche und/oder wasserquellbare Polymer; Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist; Inkontaktbringen der Nährkartonscheibe und/oder der Stützstruktur mit der wässrigen Zusammensetzung, gefolgt von Trocknen, wodurch die Nährkartonscheibe beziehungsweise die Stützstruktur, die das feste wasserlösliche Nährmedium sowie das wasserlösliche und/oder wasserquellbare Polymer umfassen, erhalten wird; und Anordnen der Filtrationsmembran, der Nährkartonscheibe und der Stützstruktur zur Filtrationseinheit.

Gemäß einer bevorzugten Ausführungsform umfasst das Herstellungsverfahren die folgenden Schritte: Bereitstellen der Filtrationsmembran; Bereitstellen einer Nährkartonscheibe, welche frei von dem wasserlöslichen Nährmedium sowie dem wasserlöslichen und/oder wasserquellbaren Polymer ist; Bereitstellen einer wässrigen Lösung des wasserlöslichen Nährmediums und des wasserlöslichen Polymers; Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist; Inkontaktbringen der Nährkartonscheibe und/oder der Stützstruktur mit der wässrigen Lösung des wasserlöslichen Nährmediums und des wasserlöslichen Polymers, gefolgt von Trocknen, wodurch die Nährkartonscheibe beziehungsweise die Stützstruktur, die das wasserlösliche Nährmedium und das wasserlösliche Polymer umfasst, erhalten wird; und Anordnen der Filtrationsmembran, der Nährkartonscheibe und der Stützstruktur zur Filtrationseinheit.

Die vorstehenden Ausführungen in Bezug auf die erfindungsgemäße Filtrationseinheit, die nachstehenden Erläuterungen in Bezug auf das Verfahren zu ihrer Herstellung sowie die nachstehenden Erläuterungen in Bezug auf das erfindungsgemäße Nachweisverfahren sind wechselseitig aufeinander anwendbar.

Die wässrige Lösung des wasserlöslichen Nährmediums (A), die Zusammensetzung, enthaltend das wasserlösliche und/oder wasserquellbare Polymer (B) und die wässrige Zusammensetzung des wasserlöslichen Nährmediums und des wasserlöslichen und/oder wasserquellbaren Polymers (AB) unterliegen keiner besonderen Einschränkung.

Erfindungsgemäß können die Zusammensetzungen (B) bzw. (AB) ein wasserlösliches Polymer, ein wasserquellbares Polymer oder sowohl ein wasserlösliches Polymer als auch ein wasserquellbares Polymer enthalten. Gemäß einer Ausführungsform der vorliegenden Erfindung sind die Zusammensetzungen (B) bzw. (AB) wässrige Lösungen, wobei das Polymer ein wasserlösliches Polymer ist.

Die Zusammensetzung (B) beziehungsweise (AB) weist vorzugsweise einen Gehalt des wasserlöslichen und/oder wasserquellbaren Polymers von 10 bis 120 g/L, besonders bevorzugt 50 bis 100 g/L auf. Innerhalb dieser Bereiche können die Zusammensetzungen (B) bzw. (AB) mit optimaler Verarbeitbarkeit bereitgestellt werden. Zudem ist es bevorzugt, dass das Massenverhältnis (wasserlösliches Nährmedium / wasserlösliches und/oder wasserquellbares Polymer) von 1/30 bis 6/1, vorzugsweise von 1/20 bis 3/1, beträgt. In diesen Bereichen wird im Filtrationsschritt des erfindungsgemäßen Nachweisverfahrens nur ein relativ geringer Anteil des Nährmediums ausgewaschen und es steht folglich im Inkubationsschritt für die gegebenenfalls vorhandenen Mikroorganismen ausreichend Nährmedium zur Verfügung.

Der Schritt des Inkontaktbringens der Nährkartonscheibe, welche frei von dem wasserlöslichen Nährmedium sowie dem wasserlöslichen und/oder wasserquellbaren Polymer ist, (im Folgenden auch Nährkartonscheibenrohling genannt) und/oder der Stützstruktur, welche frei von dem wasserlöslichen Nährmedium sowie dem wasserlöslichen und/oder wasserquellbaren Polymer ist, (im Folgenden auch Stützstrukturrohling genannt) mit der wässrigen Lösung des Nährmediums (A) bzw. der wässrigen Zusammensetzung (AB) und der Schritt des Inkontaktbringens des Nährkartonscheibenrohlings und/oder des Stützstrukturrohlings, der das Nährmedium umfasst, mit der Zusammensetzung (B), unterliegen keiner besonderen Einschränkung. Das Inkontaktbringen kann beispielsweise durch Eintauchen des Nährkartonscheibenrohlings/des Stützstrukturrohlings in die jeweilige Lösung/Zusammensetzung erfolgen. Daneben ist es möglich, die jeweilige Lösung/Zusammensetzung auf den Rohling aufzusprühen. Derartige Verfahren sind dem Fachmann hinlänglich bekannt und entsprechen im Grundsatz dem Verfahren zur Herstellung gewöhnlicher NKS, abgesehen von der Gegenwart des wasserlöslichen und/oder wasserquellbaren Polymers.

Die Zusammensetzung (B) bzw. (AB) kann als Lösung vorliegen. Daneben kann die Zusammensetzung (B) bzw. (AB) als Suspension vorliegen, insbesondere wenn ein wasserquellbares Polymer verwendet wird. Beispiele hierfür sind vernetztes Polyvinylpyrrolidon, vernetztes Polyvinylacetat, Polyacrylat, Polymethacrylat, Acrylat-Methacrylat-Copolymer, sowie Gemische daraus. Eine derartige Suspension ist vorzugsweise eine wässrige Suspension. "Wässrig" bedeutet in diesem Zusammenhang, dass Wasser enthalten ist, wobei das Wasser vorzugsweise mindestens 50 Gew.-%, insbesondere bevorzugt mindestens 80 Gew.-% der Zusammensetzung ausmacht.

Sofern die Zusammensetzung (B) bzw. (AB) als (wässrige) Suspension des wasserquellbaren Polymers vorliegt, beträgt die mittlere Teilchengröße der suspendierten Polymerteilchen vorzugsweise 0,1 µm bis 40 µm. Die mittlere Teilchengröße der suspendierten Teilchen des wasserquellbaren Polymers kann beispielsweise mittels Laserlichtbeugung gemäß ISO 13320:2020-01 bestimmt werden. Geeignet hierfür ist beispielsweise eine Vorrichtung vom Typ HELOS/BR der Firma Sympatec GmbH. Ebenso geeignet ist eine Vorrichtung vom Typ HELOS/KR bzw. vom Typ HELOS/KR-Vario der Firma Sympatec GmbH.

Der Nährkartonscheibenrohling weist eine poröse Struktur auf, welche beispielsweise eine schwammartige Struktur oder eine faserförmige Struktur sein kann. Der Nährkartonscheibenrohling schließt vorzugsweise ein Faservlies ein, wobei das Faservlies besonders bevorzugt aus Cellulosefasern aufgebaut ist. Die mittlere Porengröße des Nährkartonscheibenrohlings kann mittels Porometrie, zum Beispiel Kapillarfluss-Porometrie, bestimmt werden. Geeignet hierfür ist beispielsweise ein Porometer vom Typ "POROLUX^{™}500" der Firma POROMETER NV.

Gemäß einer bevorzugten Ausführungsform beträgt die mittlere Porengröße des Nährkartonscheibenrohlings mindestens das 10-fache, vorzugsweise das 50-fache, insbesondere bevorzugt das 100-fache der mittleren Teilchengröße der suspendierten Teilchen des wasserquellbaren Polymers der Zusammensetzung (B) bzw. (AB). Dadurch kann gewährleistet werden, dass die suspendierten Polymerteilchen ungehindert in den Nährkartonscheibenrohling eindringen können. Aus diesem Grund ist weiterhin bevorzugt, dass die mittlere Porengröße des Nährkartonscheibenrohlings mindestens 5 µm, vorzugsweise mindestens 10 µm, besonders bevorzugt mindestens 15 µm, beträgt.

Sofern das erfindungsgemäße Herstellungsverfahren eine Filtrationseinheit betrifft, in der die NKS frei von Nährmedium und wasserlöslichem/-quellbarem Polymer sein soll, ist der Nährkartonscheibenrohling mit der NKS identisch.

Der Stützstrukturrohling weist ein Trägerelement und eine Seitenwand auf, wie vorstehend für die Stützstruktur beschrieben. Der Stützstrukturrohling entspricht im Wesentlichen der vorstehend erwähnten Stützstruktur, abgesehen davon, dass der Stützstrukturrohling noch frei von dem Polymer und dem Nährmedium ist. Sofern das erfindungsgemäße Herstellungsverfahren eine Filtrationseinheit mit Stützstruktur betrifft, in der die Stützstruktur frei von Nährmedium und wasserlöslichem/-quellbarem Polymer sein soll, ist der Stützstrukturrohling mit der Stützstruktur identisch.

Im Schritt des Inkontaktbringens kann der ganze Stützstrukturrohling oder nur ein Teil desselben mit der jeweiligen Zusammensetzung/Lösung in Kontakt gebracht werden. Beispielsweise kann nur das Trägerelement oder nur die Seitenwand in Kontakt gebracht werden. Von der Seitenwand wird vorzugsweise nur die Innenseite dem jeweiligen Schritt des Inkontaktbringens unterzogen, unabhängig davon, ob auch das Trägerelement dem Schritt des Inkontaktbringens unterzogen wird oder nicht.

Erfindungsgemäß kann auf verschiedenen Wegen erreicht werden, dass die Nährkartonscheibe beziehungsweise die Stützstruktur der Filtrationseinheit ein wasserquellbares Polymer umfasst.

Zum einen kann die Zusammensetzung (B) bzw. (AB) mit einem wasserquellbaren Polymer bereitgestellt werden, insbesondere, wenn das wasserquellbare Polymer Polyacrylat, Polymethacrylat, Acrylat-Methacrylat-Copolymer oder ein Gemisch daraus ist. In diesem Fall ist bevorzugt, dass die Zusammensetzung (B) bzw. (AB) als Suspension vorliegt. Allerdings ist es ebenso möglich, die Zusammensetzung (B) als Lösung bereitzustellen. In diesem Fall wird für das wasserquellbare Polymer ein geeignetes, beispielsweise organisches, Lösungsmittel verwendet. Das Lösungsmittel für das wasserquellbare Polymer ist vorzugsweise ein Nichtlösungsmittel für das Nährmedium.

Zum anderen kann die Zusammensetzung ein wasserlösliches Polymer enthalten, aus dem in einem späteren Schritt ein wasserquellbares Polymer erzeugt wird. Zu hierfür geeigneten Polymeren zählen Polyvinylpyrrolidon und Polyvinylacetat. Somit ist bevorzugt, dass die Zusammensetzung (B) beziehungsweise (AB) als wässrige Lösung eines (zunächst) wasserlöslichen Polymers bereitgestellt wird und nach dem Inkontaktbringen mit der Zusammensetzung (B) beziehungsweise (AB) ein Vernetzungsschritt durchgeführt wird, um aus dem zunächst wasserlöslichen Polymer ein wasserquellbares Polymer zu erzeugen. Geeignete Methoden zum Vernetzen sind dem Fachmann bekannt. Beispielsweise kann durch Einstrahlen von UV-Strahlung vernetzt werden. Das in dieser bevorzugten Ausführungsform bereitgestellte zunächst wasserlösliche Polymer kann ein vorvernetztes (teilweise vernetztes) Polymer sein, wodurch der Vernetzungsschritt nach dem Inkontaktbringen besonders schnell und ohne übermäßiges Bestrahlen der Nährkartonscheibe beziehungsweise der Stützstruktur erfolgen kann.

Der beziehungsweise die Trocknungsschritte des erfindungsgemäßen Verfahrens unterliegen keiner besonderen Einschränkung. Das Trocknen dient dazu, das Lösungsmittel der entsprechenden Lösung zu entfernen, so dass die verbleibenden Feststoffe (festes Nährmedium bzw. Polymer) zurückbleibt. Das Trocknen erfolgt vorzugsweise bei Normaldruck (10⁵ Pa) und einer Temperatur von 30°C bis 250°C, vorzugsweise 80°C bis 200°C, besonders bevorzugt 90°C bis 150°C, insbesondere bevorzugt 100°C bis 130°C. Durch eine Trocknungstemperatur in diesem Bereich erfolgt die Trocknung schnell, ohne den Nährkartonscheiben- beziehungsweise Stützstrukturrohling, das Polymer oder das Nährmedium durch zu hohe Wärmeeinwirkung in unerwünschter Weise zu beeinflussen.

In einem weiteren Aspekt betrifft die vorliegende Erfindung ein Verfahren zum Nachweis von Mikroorganismen in einem Fluid, welches die folgenden Schritte umfasst: Bereitstellen der erfindungsgemäßen Filtrationseinheit; Filtrieren des Fluids durch die Filtrationseinheit; Inkubieren der Filtrationseinheit; und Auswerten der Filtrationseinheit nach dem Inkubieren. Es ist besonders bevorzugt, dass das erfindungsgemäße Verfahren aus diesen Schritten besteht. Daneben ist bevorzugt, dass zwischen Filtrieren und Inkubieren keine weiteren Schritte durchgeführt werden. Das erfindungsgemäße Nachweisverfahren umfasst vorzugsweise keinen Schritt, in dem Nährmedium zugegeben wird.

Erfindungsgemäß unterliegen die Mikroorganismen keiner besonderen Einschränkung. Grundsätzlich können mit dem erfindungsgemäßen Verfahren alle der in der Trinkwasserverordnung der Bundesrepublik Deutschland (siehe Bundesgesetzblatt Jahrgang 2011 Teil I Nr. 21, ausgegeben zu Bonn am 11. Mai 2011) genannten Mikroorganismen nachgewiesen werden. Besonders eignet sich das erfindungsgemäße Nachweisverfahren für in Flüssigkeiten enthaltene Bakterien und/oder Pilze. Die Bakterien können beispielsweise *Escherichia coli, Pseudomonas aeruginosa, Staphylococcus aureus,* und/oder *Bacillus subtilis* sein. Pilze, welche mit dem erfindungsgemäßen Verfahren nachgewiesen werden können, umfassen beispielsweise Hefen wie *Saccharomyces cerevisiae* und *Candida albicans* und/oder Schimmelpilze wie *Aspergillus brasiliensis.*

Erfindungsgemäß wird unter einem "Fluid" ein fließfähiges Stoffgemisch verstanden. Dazu zählen beispielsweise Gase, Aerosole, Lösungen, Suspensionen und Emulsionen. Vorzugsweise umfasst das Fluid eine flüssige Phase. Beispiele für derartige Fluide sind Suspensionen, Emulsionen und Lösungen. Besonders bevorzugt ist das Fluid eine Lösung. Konkrete Beispiele für Fluide, die mit dem erfindungsgemäßen Nachweisverfahren untersucht werden können, sind Getränke wie Bier, Wein, Fruchtsaft, Milch, Trink- bzw. Leitungswasser, sowie Kosmetika in fluider Form.

Der Schritt des Inkubierens unterliegt erfindungsgemäß keiner besonderen Einschränkung. Die Inkubationsbedingungen werden in Abhängigkeit vom Mikroorganismus, welcher nachgewiesen werden soll, gewählt. Entsprechend geeignete Bedingungen sind dem Fachmann hinlänglich bekannt. Der Schritt des Inkubierens kann beispielsweise unter Normaldruck bei einer relativen Luftfeuchte von 0 bis 100%, vorzugsweise 10 bis 90%, besonders bevorzugt 30 bis 70%, sowie unabhängig davon vorzugsweise bei einer Temperatur von 5°C bis 100°C, besonders bevorzugt 5°C bis 90°C, insbesondere bevorzugt 15°C bis 60°C, noch bevorzugter 25°C bis 35°C, erfolgen. Auch die Dauer des Inkubationsschritts unterliegt keiner besonderen Einschränkung und beträgt beispielsweise 10 Minuten bis 7 Tage, vorzugsweise 1 bis 72 Stunden, besonders bevorzugt 6 bis 48 Stunden, noch bevorzugter 12 bis 36 Stunden. Geeignete Inkubationsbedingungen für spezifische Mikroorganismen sind in der vorstehend erwähnten Trinkwasserverordnung aufgeführt.

Der Schritt des Auswertens der Filtrationseinheit nach dem Inkubieren unterliegt keiner besonderen Einschränkung und kann so wie in dem eingangs erwähnten klassischen Membranfilterverfahren beziehungsweise gemäß der Biosart^{®}-Technologie erfolgen. Beispielsweise kann das Auswerten durch Zählen der durch die im Inkubationsschritt gebildeten Kolonien erfolgen. Die Kolonien können von Hand oder mit Hilfe eines automatischen Verfahrens gezählt werden (quantitative Auswertung). Alternativ dazu kann der Schritt des Auswertens auch lediglich in einem qualitativen Auswerten erfolgen. Bei einer qualitativen Auswertung wird lediglich festgestellt, ob eine oder mehrere Kolonien vorliegen oder nicht.

In einer bevorzugten Ausführungsform des vorstehend genannten Verfahrens liegt das Nährmedium während des gesamten vorstehend definierten Verfahrens in dem Hohlraum der Filtrationseinheit vor.

Das Volumen des Fluids, welches durch die Filtrationseinheit filtriert wird, unterliegt keiner besonderen Einschränkung. Vorzugsweise beträgt das Volumen 1 mL bis 10 L, besonders bevorzugt 10 mL bis 500 mL, noch bevorzugter 50 mL bis 300 mL, noch mehr bevorzugt 75 bis 150 mL, beispielsweise 100 mL. Bei einem Volumen des filtrierten Fluids im vorstehenden Bereich liegt ein günstiges Gleichgewicht zwischen ausreichender Probenmenge und einem weitgehend unterdrückten Auswaschen des Nährmediums aus der Nährkartonscheibe vor.

Die Figur zeigt:
Fig. 1: Beispielhaft dargestellte Filtrationseinheit zum Nachweis von Mikroorganismen in einem Fluid in Form von drei verschiedenen Ausführungsformen A, B und C.

Die vorliegende Erfindung wird weiter durch die nachstehenden Beispiele erläutert, ohne darauf beschränkt zu sein.

### Beispiele

### Beispiel 1

Zwei Filtrationseinheiten FE-1 und FE-2 mit PVP K90 (FE-1) bzw. mit PVP K120 (FE-2) wurden wie folgt hergestellt: Das Nährmedium (Tryptic Soy Broth (TSB) von der Firma Merck) wurde entsprechend den Herstellerangaben in Wasser gelöst. In der erhaltenen Lösung wurde PVP K90 (FE-1) bzw. PVP K120 (FE-2) in einer Menge von jeweils 50 g/L und unter Rühren und Erwärmen gelöst. Anschließend wurde das jeweilige Nährmedium für 15 min bei 121°C autoklaviert. Cellulose-Pads mit einem Durchmesser von 43 mm und einer Dicke von 1,4 mm wurden mit der jeweiligen autoklavierten Lösung getränkt, bis die Lösung vollständig von dem Pad aufgenommen wurde. Nach Abtropfen wurden die Pads bei 50°C über Nacht im Trockenschrank unter Belüftung getrocknet. Danach wurden die Pads zusammen mit je einer Cellulosenitrat-Filtrationsmembran mit einem Durchmesser von 47 mm in jeweils einen Biosart^{®}-Monitor von der Firma Sartorius Stedim Biotech GmbH (Stützstruktur) einbaut. Hierzu wurde das Pad zunächst auf die Basis (Trägerelement) des Biosart^{®}-Monitors gelegt. Auf diese Anordnung wurde jeweils eine Cellulosenitrat-Filtrationsmembran platziert und schließlich wurden jeweils der Biosart^{®}-Zylinder (Seitenwand) sowie -Deckel aufgesetzt.

Zum Vergleich wurde eine Filtrationseinheit FE-X entsprechend FE-1 und FE-2 hergestellt, mit der Maßgabe, dass PVP K90 bzw. PVP K120 weggelassen wurden.

Durch die Filtrationseinheiten mit PVP K90 (Polyvinylpyrrolidon mit einem K-Wert nach Fikentscher von 90) und PVP K120 (Polyvinylpyrrolidon mit einem K-Wert nach Fikentscher von 120) wurden 100 mL Wasser filtriert. Aus der Gewichtsdifferenz der jeweiligen Filtrationseinheit vor und nach dem Filtrieren ergab sich, dass bei der Filtrationseinheit mit PVP K90 80% des Nährmediums und bei der Filtrationseinheit mit PVP K120 65% des Nährmediums ausgewaschen wurden. Im Gegensatz dazu wurden bei einer Vergleichsfiltrationseinheit, deren Nährkartonscheibe lediglich das Nährmedium, jedoch kein wasserlösliches Polymer enthielt, 100% des Nährmediums ausgewaschen.

### Beispiel 2

Entsprechend dem Herstellungsverfahren für FE-1 und FE-2 wurden zwei Filtrationseinheiten FE-3 und FE-4 hergestellt. Jeweils ein Cellulose-Pad wurde, wie vorstehend beschrieben, in 45 g/L wässrigem TSB (Tryptic Soy Broth)-Medium mit entweder 5 Gew.-% (50 g/L; FE-3) bzw. 10 Gew.-% (100 g/L; FE-4) PVP K90 getränkt und über Nacht bei 50°C getrocknet. Anschließend erfolgte jeweils das Verbauen zusammen mit der Cellulosenitrat-Filtrationsmembran in jeweils eine Biosart^{®}-Einheit.

Es wurde das Wachstum von *P. aeruginosa* auf den Filtrationseinheiten FE-3 bzw. FE-4 nach erfindungsgemäßer Verwendung der Filtrationseinheiten untersucht. Das verwendete Inokulum wurde auf 100 CFU (colony forming units) *P. aeruginosa* eingestellt, in eine 20 mL-Vorlage (0,9 Gew.-%-ige NaCl-Lösung) eingebracht und durch FE-3 bzw FE-4 filtriert. Als Kontrolle diente ein Ausstrich des auf 100 CFU eingestellten Inokulums auf TSA (Tryptic Soy Agar). Die Inkubation erfolgte jeweils bei 30-35°C für 18-24 h. Als Ergebnis wurde im Vergleich zum Ausstrich auf TSA eine Wiederfindung von *P*. *aeruginosa* von 95% beim 5% PVP-Ansatz sowie 88% beim 10% PVP-Ansatz erreicht.

### Bezugszeichenliste

- 1: Erster reversibler Deckel
- 2: Einlass der Filtrationseinheit
- 3: Hohlraum
- 4: Seitenwand
- 5: Filtrationsmembran
- 6A: Nährkartonscheibe (NKS)
- 6B: Kartonscheibe
- 7: Trägerelement
- 8: Auslass der Filtrationseinheit
- 9: Zweiter reversibler Deckel
- 10: Faservlies
- 11: Pore (offen)
- 12: Nährmedium
- 13: Wasserlösliches und/oder wasserquellbares Polymer

## Patentansprüche

1. Filtrationseinheit, umfassend eine Filtrationsmembran, eine Nährkartonscheibe und eine Stützstruktur, wobei zumindest eine von der Nährkartonscheibe und der Stützstruktur ein festes wasserlösliches Nährmedium sowie ein wasserlösliches und/oder wasserquellbares Polymer umfasst,
wobei die Nährkartonscheibe, sofern sie das feste wasserlösliche Nährmedium sowie das wasserlösliche und/oder wasserquellbare Polymer umfasst, ein Faservlies aufweist, und das feste wasserlösliche Nährmedium sowie das wasserlösliche und/oder wasserquellbare Polymer entweder
a) die Fasern des Faservlieses teilweise oder vollständig umhüllen, ohne die Poren des Faservlieses zu verschließen, oder
b) als Teilchen zwischen den Fasern verteilt sind, und
wobei die Stützstruktur ein Trägerelement und eine Seitenwand, welche einen Hohlraum definieren, aufweist, die Nährkartonscheibe und die Filtrationsmembran in dem Hohlraum angeordnet sind und die Nährkartonscheibe zwischen der Filtrationsmembran und dem Trägerelement angeordnet ist, und das feste wasserlösliche Nährmedium sowie das wasserlösliche und/oder wasserquellbare Polymer, sofern von der Stützstruktur umfasst, auf das Trägerelement und/oder die Seitenwand aufgebracht sind.

2. Filtrationseinheit nach Anspruch 1, wobei
das wasserlösliche Polymer aus der Gruppe, bestehend aus Polyvinylpyrrolidon, Gelatine, Agarose, sowie Gemischen daraus ausgewählt ist und
das wasserquellbare Polymer aus der Gruppe, bestehend aus vernetztem Polyvinylpyrrolidon, vernetztem Polyvinylacetat, Polyacrylat, Polymethacrylat, Acrylat-Methacrylat-Copolymer, sowie Gemischen daraus ausgewählt ist.

3. Filtrationseinheit nach Anspruch 1 oder 2, wobei die Nährkartonscheibe ein Faservlies einschließt.

4. Filtrationseinheit nach einem der Ansprüche 1 bis 3,
wobei die Nährkartonscheibe das feste wasserlösliche Nährmedium sowie das wasserlösliche und/oder wasserquellbare Polymer umfasst.

5. Filtrationseinheit nach einem der Ansprüche 1 bis 4,
wobei die Stützstruktur das feste wasserlösliche Nährmedium sowie das wasserlösliche und/oder wasserquellbare Polymer umfasst.

6. Filtrationseinheit nach einem der Ansprüche 1 bis 5,
wobei die Seitenwand und das Trägerelement jeweils in Form eines beidseitig offenen Zylinders vorliegen, welche jeweils an einer der offenen Seiten einen Einlass und an der gegenüberliegenden offenen Seite des Einlasses einen Auslass aufweisen,
wobei sich der Einlass des Trägerelements in Richtung des Hohlraums befindet und, ausgehend von der Nährkartonscheibe, der Einlass der Seitenwand in Richtung der Filtrationsmembran befindet,
wobei der Einlass des Trägerelements dem Auslass der Seitenwand entspricht, wobei der Einlass der Seitenwand dem Einlass der Filtrationseinheit und der Auslass des Trägerelements dem Auslass der Filtrationseinheit entspricht, und wobei ein zu filtrierendes Fluid durch den Einlass der Filtrationseinheit in die Filtrationseinheit eintritt, die Filtrationsmembran, Nährkartonscheibe und Stützstruktur passiert und an dem Auslass der Filtrationseinheit filtriert austritt.

7. Filtrationseinheit nach einem der Ansprüche 1 bis 6, wobei die Filtrationsmembran in ihren Randbereichen fluiddicht mit der Stützstruktur verbunden ist.

8. Filtrationseinheit nach einem der Ansprüche 1 bis 7, wobei die Filtrationseinheit in steriler Form vorliegt.

9. Verfahren zur Herstellung einer Filtrationseinheit nach einem der Ansprüche 1 bis 8, welches die folgenden Schritte umfasst:
Bereitstellen der Filtrationsmembran;
Bereitstellen einer Nährkartonscheibe, welche frei von dem wasserlöslichen Nährmedium sowie dem wasserlöslichen und/oder wasserquellbaren Polymer ist;
Bereitstellen einer wässrigen Lösung des wasserlöslichen Nährmediums;
Bereitstellen einer Zusammensetzung, enthaltend das wasserlösliche und/oder wasserquellbare Polymer;
Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist;
Inkontaktbringen der Nährkartonscheibe und/oder der Stützstruktur mit der wässrigen Lösung des wasserlöslichen Nährmediums, gefolgt von Trocknen, wodurch eine Nährkartonscheibe und/oder eine Stützstruktur, die das Nährmedium umfasst, erhalten wird;
Inkontaktbringen der Nährkartonscheibe und/oder der Stützstruktur, die das Nährmedium umfasst, mit der Zusammensetzung, enthaltend das wasserlösliche und/oder wasserquellbare Polymer, gefolgt von Trocknen, wodurch die Nährkartonscheibe beziehungsweise die Stützstruktur, die das feste wasserlösliche Nährmedium sowie das wasserlösliche und/oder wasserquellbare Polymer umfassen, erhalten wird; und
Anordnen der Filtrationsmembran, der Nährkartonscheibe und der Stützstruktur zur Filtrationseinheit.

10. Verfahren zur Herstellung einer Filtrationseinheit nach einem der Ansprüche 1 bis 8, welches die folgenden Schritte umfasst:
Bereitstellen der Filtrationsmembran;
Bereitstellen einer Nährkartonscheibe, welche frei von dem wasserlöslichen Nährmedium sowie dem wasserlöslichen und/oder wasserquellbaren Polymer ist;
Bereitstellen einer wässrigen Zusammensetzung, enthaltend das wasserlösliche Nährmedium und das wasserlösliche und/oder wasserquellbare Polymer;
Bereitstellen einer Stützstruktur, welche frei von dem wasserlöslichen Nährmedium sowie dem wasserlöslichen und/oder wasserquellbaren Polymer ist und ein Trägerelement und eine Seitenwand, welche einen Hohlraum definieren, aufweist;
Inkontaktbringen der Nährkartonscheibe und/oder der Stützstruktur mit der wässrigen Zusammensetzung, gefolgt von Trocknen, wodurch die Nährkartonscheibe beziehungsweise die Stützstruktur, die das feste wasserlösliche Nährmedium sowie das wasserlösliche und/oder wasserquellbare Polymer umfassen, erhalten wird; und
Anordnen der Filtrationsmembran, der Nährkartonscheibe und der Stützstruktur zur Filtrationseinheit.

11. Verfahren zum Nachweis von Mikroorganismen in einem Fluid, welches die folgenden Schritte umfasst:
Bereitstellen einer Filtrationseinheit nach einem der Ansprüche 1 bis 8;
Filtrieren des Fluids durch die Filtrationseinheit;
Inkubieren der Filtrationseinheit; und
Auswerten der Filtrationseinheit nach dem Inkubieren.

12. Verfahren nach Anspruch 11, wobei der Schritt des Filtrierens des Fluids umfasst, dass das Fluid durch einen Einlass der Filtrationseinheit in die Filtrationseinheit eintritt, die Filtrationsmembran, Nährkartonscheibe und Stützstruktur passiert und an einem Auslass der Filtrationseinheit filtriert austritt.

13. Verfahren nach Anspruch 11 oder 12,
wobei der Schritt des Filtrierens des Fluids umfasst, dass das Nährmedium in dem Hohlraum der Filtrationseinheit vorliegt.

## Claims

1. Filtration unit, comprising a filtration membrane, a nutrient pad and a support structure, wherein at least one from the nutrient pad and the support structure comprises a solid, water-soluble growth medium and a water-soluble and/or water-swellable polymer,
wherein the nutrient pad, insofar as it comprises the solid, water-soluble growth medium and the water-soluble and/or water-swellable polymer, includes a fibrous web, and the solid, water-soluble growth medium and the water-soluble and/or water-swellable polymer either
a) partially or completely encase the fibers of the fibrous web, without closing the pores of the fibrous web, or
b) are distributed as particles between the fibers, and
wherein the support structure has a carrier element and a lateral wall that define a cavity, the nutrient pad and the filtration membrane are arranged in the cavity, and the nutrient pad is arranged between the filtration membrane and the carrier element, and the solid, water-soluble growth medium and the water-soluble and/or water-swellable polymer, insofar as comprised by the support structure, are applied to the carrier element and/or the lateral wall.

2. Filtration unit according to claim 1, wherein
the water-soluble polymer is selected from the group consisting of polyvinylpyrrolidone, gelatin, agarose, and mixtures thereof, and
the water-swellable polymer is selected from the group consisting of crosslinked polyvinylpyrrolidone, crosslinked polyvinyl acetate, polyacrylate, polymethacrylate, acrylate-methacrylate copolymer, and mixtures thereof.

3. Filtration unit according to claim 1 or 2, wherein the nutrient pad includes a fibrous web.

4. Filtration unit according to any one of claims 1 to 3,
wherein the nutrient pad comprises the solid, water-soluble growth medium and the water-soluble and/or water-swellable polymer.

5. Filtration unit according to any one of claims 1 to 4,
wherein the support structure comprises the solid, water-soluble growth medium and the water-soluble and/or water-swellable polymer.

6. Filtration unit according to any one of claims 1 to 5,
wherein the lateral wall and the carrier element are each in the form of a cylinder open at both ends, each of which has an inlet at one of the open ends and an outlet at the open end opposite the inlet,
wherein the inlet of the carrier element is situated in the direction of the cavity and, starting from the nutrient pad, the inlet of the lateral wall is situated in the direction of the filtration membrane,
wherein the inlet of the carrier element corresponds to the outlet of the lateral wall,
wherein the inlet of the lateral wall corresponds to the inlet of the filtration unit and the outlet of the carrier element corresponds to the outlet of the filtration unit, and
wherein a fluid to be filtered enters the filtration unit through the inlet of the filtration unit, passes through the filtration membrane, nutrient pad and support structure, and exits in a filtered state at the outlet of the filtration unit.

7. Filtration unit according to any one of claims 1 to 6, wherein the edge regions of the filtration membrane are fluid-tightly connected to the support structure.

8. Filtration unit according to any one of claims 1 to 7, wherein the filtration unit is in sterile form.

9. Method for producing a filtration unit according to any one of claims 1 to 8, comprising the following steps:
providing the filtration membrane;
providing a nutrient pad which is free of the water-soluble growth medium and the water-soluble and/or water-swellable polymer;
providing an aqueous solution of the water-soluble growth medium;
providing a composition containing the water-soluble and/or water-swellable polymer;
providing a support structure which is free of the water-soluble growth medium and the water-soluble and/or water-swellable polymer and which has a carrier element and a lateral wall that define a cavity;
bringing the nutrient pad and/or the support structure into contact with the aqueous solution of the water-soluble growth medium, followed by drying, thereby yielding a nutrient pad and/or a support structure which comprises the growth medium;
bringing the nutrient pad and/or the support structure which comprises the growth medium into contact with the composition containing the water-soluble and/or water-swellable polymer, followed by drying, thereby yielding the nutrient pad and/or the support structure which comprise the solid, water-soluble growth medium and the water-soluble and/or water-swellable polymer; and
arranging the filtration membrane, the nutrient pad and the support structure to form the filtration unit.

10. Method for producing a filtration unit according to any one of claims 1 to 8, comprising the following steps:
providing the filtration membrane;
providing a nutrient pad which is free of the water-soluble growth medium and the water-soluble and/or water-swellable polymer;
providing an aqueous composition containing the water-soluble growth medium and the water-soluble and/or water-swellable polymer;
providing a support structure which is free of the water-soluble growth medium and the water-soluble and/or water-swellable polymer and which has a carrier element and a lateral wall that define a cavity;
bringing the nutrient pad and/or the support structure into contact with the aqueous composition, followed by drying, thereby yielding the nutrient pad and/or the support structure which comprises the solid, water-soluble growth medium and the water-soluble and/or water-swellable polymer; and
arranging the filtration membrane, the nutrient pad and the support structure to form the filtration unit.

11. Method for detecting microorganisms in a fluid, comprising the following steps:
providing a filtration unit according to any one of claims 1 to 8;
filtering the fluid through the filtration unit;
incubating the filtration unit; and
evaluating the filtration unit after the incubation.

12. Method according to claim 11, wherein the step of filtering the fluid comprises the fluid entering the filtration unit through an inlet of the filtration unit, passing through the filtration membrane, nutrient pad and support structure, and exiting in a filtered state at an outlet of the filtration unit.

13. Method according to claim 11 or 12,
wherein the step of filtering the fluid comprises the growth medium being present in the cavity of the filtration unit.

## Revendications

1. Unité de filtration, comprenant une membrane de filtration, un disque en carton nutritif et une structure de soutien, dans laquelle au moins l'un du disque en carton nutritif et de la structure de soutien comprend un milieu nutritif hydrosoluble solide ainsi qu'un polymère hydrosoluble et/ou gonflable à l'eau,
dans laquelle le disque en carton nutritif, dans la mesure où il comprend le milieu nutritif hydrosoluble solide ainsi que le polymère hydrosoluble et/ou gonflable à l'eau, présente un voile de fibres, et le milieu nutritif hydrosoluble solide ainsi que le polymère hydrosoluble et/ou gonflable à l'eau soit
a) enveloppent partiellement ou totalement les fibres du voile de fibres sans obturer les pores du voile de fibres, soit
b) sont répartis sous la forme de particules entre les fibres, et
dans laquelle la structure de soutien présente un élément de support et une paroi latérale, lesquels définissent une cavité, le disque en carton nutritif et la membrane de filtration sont disposés dans la cavité et le disque en carton nutritif est disposé entre la membrane de filtration et l'élément de support, et le milieu nutritif hydrosoluble solide ainsi que le polymère hydrosoluble et/ou gonflable à l'eau, dans la mesure où ils sont entourés par la structure de soutien, sont agencés sur l'élément de support et/ou la paroi latérale.

2. Unité de filtration selon la revendication 1, dans laquelle
le polymère hydrosoluble est choisi dans le groupe constitué de polyvinylpyrrolidone, gélatine, agarose, ainsi que des mélanges de celles-ci, et
le polymère gonflable à l'eau est choisi dans le groupe constitué de polyvinylpyrrolidone réticulée, acétate de polyvinyle réticulé, polyacrylate, polyméthacrylate, copolymère d'acrylate - méthacrylate, ainsi que des mélanges de ceux-ci.

3. Unité de filtration selon la revendication 1 ou 2, dans laquelle le disque en carton nutritif inclut un voile de fibres.

4. Unité de filtration selon l'une des revendications 1 à 3,
dans laquelle le disque en carton nutritif comprend le milieu nutritif hydrosoluble solide ainsi que le polymère hydrosoluble et/ou gonflable à l'eau.

5. Unité de filtration selon l'une des revendications 1 à 4,
dans laquelle la structure de soutien comprend le milieu nutritif hydrosoluble solide ainsi que le polymère hydrosoluble et/ou gonflable à l'eau.

6. Unité de filtration selon l'une des revendications 1 à 5,
dans laquelle la paroi latérale et l'élément de support se présentent respectivement sous la forme d'un cylindre ouvert des deux côtés, qui présentent respectivement sur l'un des côtés ouverts une entrée et sur le côté ouvert opposé de l'entrée une sortie,
dans laquelle l'entrée de l'élément de support se trouve dans la direction de la cavité et, en partant du disque en carton nutritif, l'entrée de la paroi latérale se trouve dans la direction de la membrane de filtration,
dans laquelle l'entrée de l'élément de support correspond à la sortie de la paroi latérale,
dans laquelle l'entrée de la paroi latérale correspond à l'entrée de l'unité de filtration et la sortie de l'élément de support correspond à la sortie de l'unité de filtration, et dans laquelle un fluide à filtrer pénètre dans l'unité de filtration par l'entrée de l'unité de filtration, traverse la membrane de filtration, le disque en carton nutritif et la structure de soutien, et sort filtré au niveau de la sortie de l'unité de filtration.

7. Unité de filtration selon l'une des revendications 1 à 6, dans laquelle la membrane de filtration est reliée de manière étanche aux fluides à la structure de soutien dans ses zones périphériques.

8. Unité de filtration selon l'une des revendications 1 à 7, dans laquelle l'unité de filtration se présente sous une forme stérile.

9. Procédé de fabrication d'une unité de filtration selon l'une des revendications 1 à 8, qui comprend les étapes suivantes :
mise à disposition de la membrane de filtration ;
mise à disposition d'un disque en carton nutritif, lequel est exempt du milieu nutritif hydrosoluble ainsi que du polymère hydrosoluble et/ou gonflable à l'eau ;
mise à disposition d'une solution aqueuse du milieu nutritif hydrosoluble ;
mise à disposition d'une composition contenant le polymère hydrosoluble et/ou gonflable à l'eau ;
mise à disposition d'une structure de soutien, laquelle est exempte du milieu nutritif hydrosoluble ainsi que du polymère hydrosoluble et/ou gonflable à l'eau et présente un élément de support et une paroi latérale qui définissent une cavité ;
mise en contact du disque en carton nutritif et/ou de la structure de soutien avec la solution aqueuse du milieu nutritif hydrosoluble, suivie du séchage, moyennant quoi un disque en carton nutritif et/ou une structure de soutien qui comprend le milieu nutritif est obtenu ;
mise en contact du disque en carton nutritif et/ou de la structure de soutien qui comprend le milieu nutritif, avec la composition contenant le polymère hydrosoluble et/ou gonflable à l'eau, suivie du séchage, moyennant quoi le disque en carton nutritif et/ou la structure de soutien, qui comprennent le milieu nutritif hydrosoluble solide ainsi que le polymère hydrosoluble et/ou gonflable à l'eau, est obtenu ; et
agencement de la membrane de filtration, du disque en carton nutritif et de la structure de soutien sur l'unité de filtration.

10. Procédé de fabrication d'une unité de filtration selon l'une des revendications 1 à 8, qui comprend les étapes suivantes :
mise à disposition de la membrane de filtration ;
mise à disposition d'un disque en carton nutritif, lequel est exempt du milieu nutritif hydrosoluble ainsi que du polymère hydrosoluble et/ou gonflable à l'eau ;
mise à disposition d'une composition aqueuse, contenant le milieu nutritif hydrosoluble et le polymère hydrosoluble et/ou gonflable à l'eau ;
mise à disposition d'une structure de soutien, laquelle est exempte du milieu nutritif hydrosoluble ainsi que du polymère hydrosoluble et/ou gonflable à l'eau et présente un élément de support et une paroi latérale qui définissent une cavité ;
mise en contact du disque en carton nutritif et/ou de la structure de soutien avec la composition aqueuse, suivie du séchage, moyennant quoi le disque en carton nutritif et/ou la structure de soutien, qui comprennent le milieu nutritif hydrosoluble solide ainsi que le polymère hydrosoluble et/ou gonflable à l'eau, est obtenu ;
agencement de la membrane de filtration, du disque en carton nutritif et de la structure de soutien sur l'unité de filtration.

11. Procédé de détection de micro-organismes dans un fluide, qui comprend les étapes suivantes :
mise à disposition d'une unité de filtration selon l'une des revendications 1 à 8 ;
filtration du fluide par l'unité de filtration ;
incubation de l'unité de filtration ; et
évaluation de l'unité de filtration après incubation.

12. Procédé selon la revendication 11, dans lequel l'étape de filtration du fluide comprend le fait que le fluide entre dans l'unité de filtration par une entrée de l'unité de filtration, traverse la membrane de filtration, le disque en carton nutritif et la structure de soutien et sort filtré au niveau d'une sortie de l'unité de filtration.

13. Procédé selon la revendication 11 ou 12,
dans lequel l'étape de filtration du fluide comprend le fait que le milieu nutritif se présente dans la cavité de l'unité de filtration.
